# EUROPEAN PATENT APPLICATION

(11) **EP 1 502 594 A1**
(43) Date of publication of application: **02.02.2005**
(21) Application number: 03017345.4
(22) Date of filing: 31.07.2003
(51) Int. Cl.: A61K 35/78, A61P 7/02

(54) **A health care composition for decreasing the risk of thrombosis including a mixture of proanthocyanidins and sesquiterpenes**

(71) Applicant: Pynogin GmbH, 6315 Oberägeri (CH)
(72) Inventor: Lehmann, Michael, 6315 Oberägeri (CH); Burkard, Adriana, 6315 Oberägeri (CH)

(57) **Abstract**

Disclosed is a health care composition for decreasing the risk of thrombosis particularly within the lower human veins including a mixture of proanthocyanidins and sesquiterpenes, and a method for preparing such composition.

## Description

The invention refers to a health care product for decreasing the risk of thrombosis which may occur particularly within the lower human veins.

Travellers, which have to sit motionless and in restricted space upon a seat for example in an aircraft often suffer from pain in their legs which may lead in severe cases to a thrombosis (Travellers Thrombosis). A preparation including acetylsalicylic acid, which inhibits the aggregation of thrombocytes is often administered to the patients as a remedy from their pain. Such preparations, however, involve the risk of inner bleeding out of the vascular capillaries into the surrounding tissue. It is known that ugly blue spots appear easily upon the skin in case such tissue is compressed from the exterior.

It therefore has been proposed to utilise an agent for inhibiting aggregation of thrombocytes which may be extracted from the bark of the French maritime pine tree (Pinus pinaster-Ait.). Such bark extract includes among other ingredients mainly procyanidins. Extended clinical tests confirmed that a preparation on the basis of procyanidins is not only superior to acetylsalicylic acid in inhibiting aggregation but is also free of any side effects, particularly does not cause any inner bleeding. Preparation and qualities of a pine bark extract including procyanidins named in the trade as Pycnogenol are disclosed e.g. in USA Patent 5,720,956 which patent discloses also a method of controlling human platelet reactivity by Pycnogenol. For the following description it should be understood that the phrase "pine bark extract" means in essence the product Pycnogenol.

Pine bark extract had been subject of a number of studies for finding out scientifically confirmed qualities and effects on human body. Such studies are enlisted in the appending references Nos 1 - 11. Regarding venous insufficiency and oedema a double blind placebo controlled study is disclosed in reference 1. This study shows that 4 days of Pycnogenol treatment statistically and significantly reduced the leg volume increase while remaining seated, while placebo had no significant effect. The authors conclude that Pycnogenol displays a significant inhibition of oedema during hydrostatic burden.

Reference 2 has for subject a double blind placebo controlled study. Already one month of treatment with Pycnogenol relieved a statistically relevant number of patients from the typical venous insufficiency symptoms, while placebo showed no effect. Particularly the relief from pain was very efficient already after 1 month. At the end of the 2 months trial the majority of patients in the Pycnogenol group showed no signs of swelling or pain.

According to reference 3 Pycnogenol statistically and significantly improved symptoms of heaviness and swellings, and the symptoms completely disappeared in 33% (heaviness) and 88% (swellings) of patients. Placebo showed no significant benefit.

In reference 4 the efficacy of Pycnogenol for treatment of venous insufficiency was compared to another active ingredient widely used for this purpose: horse chestnut seed extract. Pycnogenol was statistically and significantly more effective for reduction of leg swelling than horse chestnut seed extract, despite the fact that the latter was given in higher dosage. Further, Pycnogenol was statistically and significantly more effective than horse chestnut seed extract for reducing the symptoms "heaviness", "cramps" and "night-time swelling".

In a study (reference 5) on oedema formation in ears and paws of rodents it was shown that the higher oligomeric procyanidins in Pycnogenol are particularly effective. These results support the theory that large procyanidins bind to proteins of defective blood capillaries to yield a lower capillary permeability (reference 6). This activity is the basis for Pycnogenol's wide-spread use for prevention and treatment of vascular disorders (reference 7).

Clinical studies with Pycnogenol (reference 8) have shown with elevated platelet activity, such as smokers and elderly, that the activation of platelets is prevented. Pycnogenol was found to dose-dependently reduce platelet activity, with statistical significance after using a single dose of at least 100 mg Pycnogenol. The authors of that study propose that simulation of nitric oxide production by Pycnogenol inhibits aggregation of platelets. Thus, Pycnogenol supports body-own mechanisms to maintain healthy platelet functions.

A single dose of 100 mg of Pycnogenol was found to be as effective for reduction of platelet activity as acetyl-salicylic acid, a substance widely used for suppression of platelet aggregation. However, acetyl-salicylic acid irreversibly inhibits the enzyme COX involved in platelet aggregation. As a result acetyl-salicylic acid dramatically increases the bleeding time causing severe adverse effects, primarily gastro-intestinal problems. Fortunately, Pycnogenol does not significantly increase bleeding time allowing a long term use for preventing of thrombotic events.

Pycnogenol has been demonstrated in various studies to be one of the most powerful natural antioxidants.

The study reference 9 carried out at the University of California showed that Pycnogenol is more powerful than vitamin C and vitamin E. Dr. Packer found that Pycnogenol is so powerful that it recycles oxidised (spent) vitamin C back to the bioactive form and protects vitamin E from oxidation. An in vitro study (reference 10) made at the University of Tokyo showed that Pycnogenol is a dramatically more powerful antioxidant than any other antioxidant tested (coenzyme Q 10, vitamin C, α-lipoic acid, vitamin E, grape seed extract) to protect fragile lipids of the eye. The clinical study according to reference 11 was carried out with 45 healthy volunteers at the University of Texas to demonstrate the increased antioxidant capacity (ORAC) in the blood following consumption of Pycnogenol. The result was a striking increase of antioxidant capacity by 40%, both after three weeks and six weeks, while the ORAC value fell back to baseline when Pycnogenol was not taken for four weeks.

The Medical Guidelines for Airline Travel, 2^{nd} edition. Volume 74 Number 5 Section 11, Supplement May 2003 states "Today's aircraft have very low cabin humidity, usually ranging from 10-20%. This is unavoidable because the air at high altitude is practically devoid of moisture. As a result, there can be a drying effect of the airway passages, the cornea (particularly under contact lenses) and the skin."

Further, a recent study conducted by the French Skin Research Centre CERIES has revealed that the ultra dry air in the cabin of an aircraft on a long haul flight (the humidity can fall below that of the Sahara) can dehydrate the skin in just three hours. Researchers have dubbed this phenomena "Hydrative Stress"

In a study by Wang in 1999 Pycnoenol was shown to improve micro-circulation of the tiny skin capillaries, supporting a better oxygen and nutrient supply, better hydration as well as an improved waste removal.
"Pycnogenol helps fighting against heart disease by inhibiting adhesion and aggregation of platelets and improving microcirculatory blood flow in human."
In: Wang,S., Tan,D., Zhao,Y., Gao,G., and Hu,L. (1999): The effect of Pycnoegnol on the microcirculation, platelet function and ischemic myocardium in patients with coronary artery diseases. European Bulletin of Drug Research, 7 (2): 19-25

Last not least reference is made to US Patent 4,698,360 titled "Plant Extract with a Proanthocyanidins Content as Therapeutic Agent having Radical Scavenger Effect (RSE) and Use Thereof" according to which the radical scavenger effect of pine bark extract is some 20 times higher than that of vitamin C. For oral administration and antioxidant effect it is recommended generally from 1.5 mg to 3 mg per day per kilogram of body weight for warm blooded animals which represents for an adult man weighing 70 kg a daily dose of about 100 to 200 mg of proanthocyanidins to be administered by unit dosed for example of 50 to 100 mg so as to obtain a radical scavenger effect in the above mentioned therapeutic indications.

One of the objects of the invention resides in creating a health care composition having improved platelet aggregation inhibiting function and being substantially free of side effects. To this end the invention provides for a health care product for decreasing the risk of thrombosis particularly within the lower human veins which includes a mixture of procyanidins and sesquiterpenes. Tests showed that adding of sesquiterpenes which may be obtained e.g. from a ginger extract, to the pine bark extract containing mainly procyanidins allows producing a preparation having improved platelet aggregation inhibiting function which is of particular importance for passengers of long haul flights in increased altitudes.

Qualities and so far known effects of the dried rhizome of Zingiber officinale Roscoe, hereinafter simply called ginger, are disclosed, apart from different documents, in the WHO Monographs on Selected Medical Plants, pages 277 - 287. So references 12 - 15 report on the prophylaxis of nausea and vomiting associated with motion sickness, supported by clinical data related to ginger. The prophylaxis of pernicious vomiting in pregnancy is subject of reference 16. The studies according to references 17 and 18 refer to the prophylaxis of seasickness. References 19 - 23 reveal results as to the treatment of dyspepsia, flatulence, colic, vomiting, diarrhoea, spasms and other stomach complaints. According to references 24 - 29 ginger may affect bleeding times and immunological parameters owing to its ability to inhibit thromboxane synthase and to act as a prostacyclin agonist.

As to antioxidant effects of ginger the authors of the study according to reference 30 investigated the effect of spice principles on scavenging of superoxide anion. The superoxide anions, as measured by nitrobluetetrazolium (NBT) reduction in xanthine-xanthine oxidase system were inhibited by superoxide dismutase, spice principles eugenol (cloves) and cuminaldehyde (cumin), antooxidants, butylated hydroxy toluene and butylated hydroxyanisole in a dose dependent manner. Zingerone (ginger) inhibited NBT reduction to a maximum of 23%. In other words, superoxide radicals were inhibited by zingerone from ginger.

According to reference 31 Cao et al studied scavenging effects of ginger on superoxide anion and hydroxyl radical. Ginger can significantly scavenge O₂- in hypoxanthinexanthine oxidase system and -OH in ultraviolet exposure of H₂O₂ system. The scavenger effects of ginger on O₂- and -OH may contribute to explaining some of the pharmacological mechanisms of this drug.

The antioxidative effect of more than 50 ethanol extracts of Chinese drugs on the air oxidation of linoleic acid was studied in reference 32. Several ethanol extracts such as glycyrrhiza uralensis, magnolia officinalis and zingiber officinale etc were found having strong antioxidative effect. Reference 33 reports that zingerone from ginger inhibited lipid peroxidation at high concentrations (greater than 150 microM).

Ginger's action against oedema and anti-inflammatory activity of ginger are demonstrated in reference 34 according to which ginger (100 mg/kg) was effective as acetyl-salicylic acid (100mg/kg) in reducing carrageenin induced oedema in rates. Similar results for the anti-inflammatory and analgesic activities of ginger were reported by reference 35. It is thought that that these anti-inflammatory actions are a result of inhibition of prostaglandin release, and hence ginger may act in a similar fashion to other non-steroidal anti-inflammatory drugs which interfere with prostaglandin release or biosynthesis.

Ginger as Carrier Herb: Ginger is also renowned for its ability to assist in the absorption of other foods, nutrients and medicines in the body.

The authors of reference 36 examined the effect of ginger rhizome on gastro-intestinal motility based on its ability to enhance charcoal meal transport in mice. Oral administrations of the actone extract of ginger (which contains volatile oils and bitter substances) at 75 mg/kg (6)-shogaol at 2,5mg/kg, or a (6)-, (8)- or (10)-gingerol at 5 mg/kg enhanced the transport of a charcoal meal. The effects of these substances were similar to or slightly weaker than those of metoclopramide and donperidone. According to reference 37 experiments were conducted to evaluate the scientific basis of the use of the trikatu group of acrids (long pepper, black pepper and ginger) in the large number of prescriptions in Ayurveda. (3H) vascine and (3H) sparteine were taken as test drugs. The results suggest that these acrids have the capacity to increase the bioavailability of certain drugs. It appears that the trikatu group of drugs increase the bioavailability either by promoting rapid absorption from the gastrointestinal tract, or by protecting the drug from being metabolised/oxidised in its first passage through the liver after being absorbed, or by a combination of these two mechanisms.

Particularly if the health care composition according to the invention includes a surplus of sesquiterpenes in relation to the proanthocyanidin containing pine bark extract.

The invention is not restricted to a utilisation of the pine bark extract from the pinus pinaster. The main agent proanthocyanidin can be found also in grape seed and cranberry seed. Therefore, the health care product according to the invention may also include mixtures of extracts of grape seed and/or cranberry seed and/or bark of the pinus pinaster.

At present, best results are obtained by the health care product according to the invention which preferably includes a mixture of 100 weight units of a 100% standardised pine bark extract from the French Maritime pine bark Pinus pinaster Ait. and 250 weight units of standardised ginger extract which includes a content of approximately 5% of the 250 weight units of gingeroles and approximately 1,5% of the 250 weight units of shagoals.

Thus, the invention represents a unique combination of standardised ginger and standardised oligometric proanthocyanidins. The invention offers the following physiological benefits:
1) Reduction of platelet aggregation: following the unique posology for vehicular long haul trips of 1 capsule for 5 days with the third capsule being taken the day of the trip and the remaining 4 capsules taken two days before the trip and two days after the trip - both procyanidins and the active ingredients of ginger reduce the possibility of platelet aggregation.
2) Inhibition of the onset of oedema for people sitting for extended periods of time: Swollen and heavy legs, ankles (lower extremities) is a common problem for people sitting for extended periods of time. Research clearly demonstrates that procyanidins significantly reduce the likelihood of the onset of oedema for people sitting for extended periods of time.
3) The standardised ginger in the medicine according to the invention is effective as an antinausea and antiemetic: Clinical studies have demonstrated that the oral administration of ginger is more effective than dimenhydrinate in preventing the gastrointestinal symptoms of kinetosis (motion sickness). Having a standardised ginger extract with a minimum 10:1 concentration to that of powered ginger means the recommended posology of the WHO Monograph of .5 g 2-4 times daily is easily met with one capsule containing the medicament according to the invention offering the equivalent of 2.5 g of powered ginger.
4) The antioxidant capacity of the invention reduces free radical damages during long haul flights: Procyanidins and ginger are both powerful antioxidants which help to reduce the damage caused by free radicals generated as a result of long haul flights. Cosmic radiation exposure is much higher for long haul flights due to the increased altitude of long haul flights as compared to short haul flights and this is particularly magnified near the respective poles. Further, research shows that the electromagnetic fields which are also pronounced on aircrafts might actually increase the half life of free radicals which both oligomeric proanthocyanidins and ginger are known to scavenge. Several studies support the possibility that magnetic fields inhibit a cell's ability to protect itself from ionising radiation.
5) Procyanidins have been demonstrated to improve the microcirculation of the blood and therefore improves the hydration of the skin which is particularly important for passengers of long haul flights where moisture levels in the cabin can be as severe as that of a desert placing passengers skin under hydrative stress
6) Research shows that ginger is an effective enhancer of the bioavailability of other nutrients and vitamins when it is present within the human gastrointestinal tract. Therefore, the standardised ginger in the medicament according to the invention actually increases the level of procyanidins to be found in the bloodstream thereby increasing the relative efficacy of the procyanidins with respect to platelet aggregation, oedema, hydration of the skin and free radical scavenging effect

An example for preparing the product according to the invention may be given: An extract of the French maritime pine bark and ginger are blended in a weight ratio of 1 : 2,5. 2340 weight units of such blend were granulated in a planetary mixer with a suspension of 11.7 weight units of Plasdone K 29 - 32 and 11.7 weight units of Aerosil 200 in 250 weight units isopropylic alcohol. The mass was wetted with additional 400 weight units isopropylic alcohol for better granulation. The wet mass was sieved through a 10.8 mm sieve and dried on hurdles at 40 - 45°C. The dry granules were sieved again through a 0.8 mm sieve. The mass was thereafter encapsulated.

In an alternative the material may be granulated and dried in a ROTO vacuum mixer and drier with inlet air of 60°C and the product temperature up to 30 - 40°C.

The standardised ginger extract is a concentrated form and an equivalent to 10 : 1 - 15 : 1 of powdered ginger. Therefore, at 10 : 1 concentration 250 mg of standardised ginger extract in each capsule is equivalent to 2500 mg of powdered ginger material satisfying the recommended posology of the WHO Monograph.
For motion sickness in adults and children older than 6 years it is recommended: 0.5 g 2-4 times daily. Dyspepsia: 2-4 g daily as powered plant material or extracts.

### References

1. Schmidtke I, Schoop W. Das hydrostatische odem und seins Beeinflussung. Swiss Med 6 (4a): 67-69,1984
2. Arcangeli P. Fitoterapia 71: 236-244, 2000
3. Petrassi C, Mastromarino A, Spartera C. Pycnogenol in chronic venous insufficiency. Phtyomedicine 7: 383-388, 2000
4. Koch, R. Comparative study of Venostasin and Pycnogenol in chronic venous insufficiency. Phytotherapy Research, 16: S1-S5.
5. Blazso G, Gabor M, Sibbel R, Rohdewald P. Anti-inflammatory and superoxide radical scavenging activities of procyanidin-containing extracts form the bark of Pinus pinaster and its fractions. Pharm Pharmacol. Lett,3: 217-220,1994
6. Rohdewald P. Pycnogenol. In: Flavanoids in health and disease. Marcel Dekker, p 405-419.1998
7. Rohdewald P. Frechn Maritime Pine bark extract (Pycnogenol), a versatile herbal supplement. Journal of Clinical Pharmacology & Therapeutics 40: 158-168.2002
8. Putter M, Grotemeyer KH, Wurthwein G, Araghi-Niknam N, Watson RR, Rohdewald P. Inhibition of smoking-induced platelet aggregation by Aspirin and Pycnogenol. Thrombosis Research 95: 155-162,1999
9. Cossins E. Lee R, Packer L, ESR studies of vitamin C regeneration, order of reactivity of natural source phytochemical preparations. Biochem Mol Biol Int 45:583-598,1998
10. Chida M, Suzuki K, Nakanishi_ueda T, Ueda T, Yasuhara H, Koide R, Armstrong D. In Vitro testing of antioxidants and biochemical endpoints in bovine retinal tissue. Ophthalmic Res 31: 407-415.1999
11. Davarj S, Kaul N, Schonlau F, Rohdewald P, Jialal I. Pycnogenol supplementation increases antioxidant capacity and has a favourable effect on lipid profile in humans, in print 2002
12. Reynolds JEF, ed. *Martindale, the extra pharmacopoeia,* 30^{th} ed. London, Pharmaceutical Press, 1993:885
13. German Commission E Monograph, Zingiberis rhizoma. *Bundesanzeiger,* 1988, 85:5 May
14. Mowrey DB, Clayson DE. Motion sickness, ginger, and psychophysics. Lancet, 1982,1:655-657
15. Holtmann S et al. The anti-motion sickness mechanism of ginger. A comparative study with placebo and dimenhydrinate. *Acta otoayngology*, 1989, 108:168-174
16. Fischer-Rasmussen W et al. Ginger treatment of hyperemesis gravidarum. *European journal of obstetrics, gynaecology and reproductive biology.* 1991. 38:19-24
17. Schmid R et al. Comparison of seven commonly used agents for prophlyaxis of seasickness. *Journal of travel medicine,* 1994, 1:203-206
18. Grontved A et al. Ginger root against seasickness . A controlled trial on the open sea. *Acta otolaryngology,* 1988, 105:45-49
19. *Standard of* ASEAN *herbal medicine*, Vol.I. Jakarta, ASEAN countries,1993
20. *Pharmacopoeia of the People's Republic of China* (English ed.). Guangzhou, Guandong Science and Technology Press, 1992.
21. *African pharmacopoeia,* Vol 1. 1^{st} ed. Lagos, Organization of African Unity, Scientific, Technical & Research Commission, 1985.
22. Farnsworth NR,ed. NAPRALERT database. Chicago, University of Illinois at Chicago, IL, March 15, 1995 production (an on-line database available directly through the University of Illinois at Chicago or through the Scientific and Technical Network (STN) of Chemical Abstracts Services)
23. German Commission E Monograph, Zingiberis rhizoma. *Bundesanzeiger,* 1988, 85:5 May
24. Srivastava KC. Aqueous extracts of onion, garlic and ginger inhibit platelet aggregation and alter arachidonic acid metabolism. *Biomedica biochimica acta,* 1984, 43:335-346
25. Backon J. Ginger: inhibition of thromboxane synthetase and stimulation of prostacyclin; relevance for medicine and psychiatry. *Medical hypotheses*, 1986,20: 271-278
26. Backon J. Ginger as an antiemetic: possible side effects due to its thromboxane synthetase activity. Anaesthesia, 1991 46: 705-706
27. Verma, S.K., J. Singh, R.Khamesra, A. Rordia.1993. Effect of ginger on platelet aggregation in man. *Indian J Med Res* 98: 240-242
28. Suekawa, M.. et al (1986). Pharmacological Studies on ginger. IV. Effect of (6) shogoal on the arachidonic cascade
29. Guh. J.H., et al (1995). Antiplatelet effect of gingerol isolated from Zingiber Officinale. *J. Pharm.Pharmacol.* 47 (4): 329-332
30. Krishnakantha. T.P. & Lokesh, B.R. (1993). Scavenging of super anions by spice principles. *Indian J. Biochem. Biophys.* 30 (2): 133-134
31. Cao, Z.F, et al. (1993). Scavenging effects on ginger superoxide anion and hydroxyl radical. *Chung Kuo Chung Yao Tsa Chih.* 18 (12): 750-751, 764
32. Zhou, Y. & Xu, R. (1992) Antioxidative effect of Chinese drugs. Chung Kuo Chung Yao Tsa Chih. 17 (6): 368-369,373
33. Reddy. A.C. & Lokesh. B.R. (1992). Studies on spice principles as antioxidants in the inhibition of lipid peroxidation of rat liver microsomes. *Mol. Cell. Biochem.* 111 (1-2): 117 -124
34. Jana, U. Chattopadhyay. RN. & Shaw, BP. (1999) Preliminary studies on anti-inflammatory activity of Zingiber officinale Rose., Vitex negundo Linn and Tinospora cordifolia (Willid) Miers in albino rates. *Indian J Pharmacol* 32, 232-233
35. Mascolo, N, Jain,R Jain, SC, and Caposso, F. (1989) Ethnopharmacologic investigation of Ginger (Zingiber Officinale). *J Ethnopharmacol* 27, 129-140
36.Yamahara J, Huang QR, Li YH, Xu L, Fujimura H. Gastrointestinal motility enhancing effect of ginger and its active constituents. *Chem Pharm Bull* (Tokyo). 1990 Feb;38 (2): 430-1
37.Atal CK, Zutshi U. Rao PG. Scientific evidence on the rold of Ayurvedic herbals on bioavailabilty of drugs. J Ethnopharmacol. 1982 Sept; 4(2): 229-32

## Claims

1. A health care composition for decreasing the risk of thrombosis particularly within the lower human veins including a mixture of proanthocyanidins and sesquiterpenes.

2. Composition according to claim 1, wherein the procyanidins are of natural origin.

3. Composition according to claim 1 or 2, wherein the sesquiterpenes are of natural origin.

4. Composition according to claim 2 or 3, wherein the procyanidins are extracted from the pine bark and/or the cranberry seed and/or the grape seed.

5. Composition according to claim 3 or 4 wherein the sesquiterpenes are extracted from the rhizome of ginger.

6. Composition according to claims 4 and 5, wherein the sesquiterpenes are included in surplus in relation to proanthocyanidins.

7. Composition according to claim 6 including a mixture of 100 weight units of a 100% standardised pine bark extract of the French maritime pine (Pinus pinaster Ait.) and 250 weight units of a standardised ginger extract which has a content of approximately 5% of the 250 weight units of gingeroles and approximately 1,5% of the 250 weight units of shagoals.

8. Method for preparing the composition according to one of the preceding claims wherein an extract of a pine bark and an extract from ginger are blended in a weight ratio of 1 : 2,5, and thereafter the blend is granulated with a wetting suspension, the wet mass is sieved, thereafter dried and filled in capsules ready for being administered orally.

9. Method according to claim 8, wherein the wetting suspension includes substantially isopropyl alcohol.
